# FASCICULE DE BREVET EUROPEEN

(11) **EP 1 856 080 B1**
(45) Date de publication et mention de la délivrance du brevet: **01.12.2010**
(21) Numéro de dépôt: 06709352.6
(22) Date de dépôt: 21.02.2006
(51) Int. Cl.: C07D 305/14

(54) **PROCEDE DE PREPARATION DU DOCETAXEL**
VERFAHREN ZUR HERSTELLUNG VON DOCETAXEL
METHOD FOR PREPARING DOCETAXEL

(30) Priorité: 24.02.2005 FR 0501882
(43) Date de publication de la demande: 21.11.2007
(73) Titulaire: Seripharm, 72650 Le Mans (FR)
(72) Inventeur: LEZE, Antoine, Paul, Gaston, F-72650 La Milesse (FR); PERION, Régis, F-72000 Le Mans (FR)
(74) Mandataire: Hirsch & Associés
(86) Numéro de dépôt international: PCT/FR2006/000386
(87) Numéro de publication internationale: WO 2006/090056

(56) Documents cités:
- WO-A-96/23780
- WO-A-2004/033442
- GUERITTE-VOEGELEIN F ET AL: "Relationships between the structure of taxol analogues and their antimitotic activity" JOURNAL OF MEDICINAL CHEMISTRY, vol. 34, no. 3, mars 1991 (1991-03), pages 992-998, XP002350804

## Description

La présente invention concerne un procédé de préparation du docétaxel à partir du paclitaxel.

Des procédés de préparation du docétaxel ont déjà été largement décrits. Cependant tous les procédés ne sont pas toujours mis en oeuvre à partir d'une matière première facilement accessible, ou ne sont pas aisément industrialisables.

Le paclitaxel est maintenant largement disponible sur le marché, ce qui, outre son intérêt thérapeutique, peut en faire une matière première intéressante, facilement accessible et d'une pureté constante et reproductible.

Dans la demande internationale WO 96/23780 a été décrit un procédé qui consiste à préparer l'amine primaire du paclitaxel et sa conversion en paclitaxel ou en d'autres dérivés de taxanes, notamment le docétaxel. Cependant, les essais ne sont jamais conduits en vue de préparer une amine primaire du 10-déacétyl paclitaxel protégé sur les fonctions hydroxy en positions -7, -10 et -2', et la conversion d'une telle amine en docétaxel protégé en positions -7, -10 et -2' ni par la suite en docétaxel n'est décrite. La mise en oeuvre du procédé décrit est difficilement transposable au niveau industriel.

Il a été maintenant trouvé, et c'est ce qui fait l'objet de la présente invention que l'on pouvait préparer le docétaxel à partir du paclitaxel en mettant en oeuvre les étapes suivantes :
a) déacétylation du paclitaxel de formule : 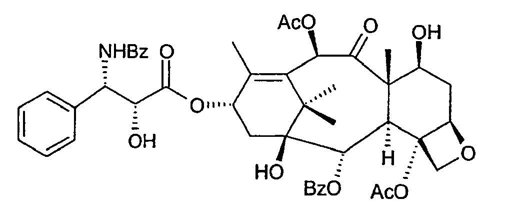
b) protection des fonctions hydroxy libres, respectivement en position -7, -10 et -2',
c) débenzoylation de l'amine en position -3' en une amine primaire dérivée de la 10-déacétylbaccatine dont les fonctions hydroxy en position -7, -10 et - 2'sont protégées,
d) fonctionnalisation de famine par un radical t.butoxycarbonyle pour obtenir un dérivé du docétaxel de formule générale : 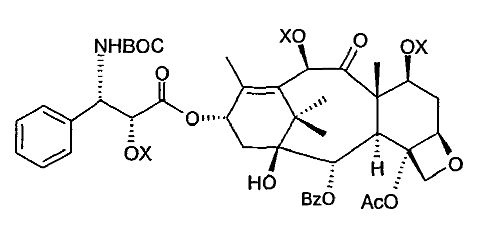 dans laquelle X représente des radicaux protecteurs ou des atomes d'hydrogène,
e) puis, le cas échéant, libération des fonctions hydroxy initialement protégées pour obtenir le docétaxel de formule :

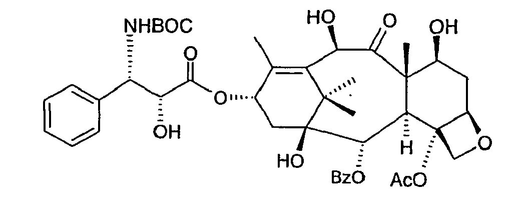

Avantageusement la déacétylation de l'étape a) s'effectue par action d'un peroxyde en milieu basique.

Le peroxyde utilisé est choisi parmi les peroxydes habituellement utilisés en milieu industriel et qui n'altèrent pas le reste de la molécule. Notamment il est choisi parmi le peroxyde d'hydrogène, le peroxyde de tert.butyle l'acide métachloroperbenzoïque ou un sel de l'acide monoperoxyphtalique. De préférence on utilise le peroxyde d'hydrogène.

La base est choisie de préférence parmi les carbonates ou les hydroxydes de métaux alcalins ou alcalino-terreux. Plus particulièrement et à titre non limitatif, la base est choisie parmi le carbonate de calcium, le bicarbonate de sodium, le carbonate de sodium, le carbonate de potassium, le carbonate de baryum, le carbonate de césium, l'hydroxyde de sodium, de potassium, de lithium, de baryum, de calcium ou de magnésium.

La réaction s'effectue dans l'eau ou dans un solvant organique comme un éther (tétrahydrofurane par exemple), un alcool (méthanol, éthanol par exemple), une cétone (acétone par exemple) ou dans un solvant chloré (par exemple le dichlorométhane, le dichloréthane, le chloroforme) à une température comprise entre 0 et 50°C, de préférence à une température comprise entre 20 et 25°C. On opère avantageusement sous atmosphère d'azote.

La protection des radicaux hydroxy respectivement en -7, -10 et -2' par des radicaux protecteurs X s'effectue selon toutes les méthodes connues et utilisées dans la chimie des taxanes et pour lesquelles la mise en place des radicaux protecteurs et leur élimination n'altère pas le reste de la molécule. Notamment, les radicaux protecteurs d'hydroxy peuvent être choisis parmi les radicaux silylés comme les groupements trialkylsilyle, dialkylarylsilyle, alkyldiarylsilyle ou triarylsilyle dans lesquelles les radicaux alkyles contiennent 1 à 4 C en chaîne droite ou ramifiée et aryle est de préférence phényle, par exemple des radicaux triéthylsilyle ou triméthylsilyle, ou des radicaux chlorés comme les radicaux trichloroalkyloxycarbonyle par exemple trichloréthoxycarbonyle notamment trichloro-2,2,2 éthoxycarbonyle ou trichlorométhylpropoxycarbonyle notamment (trichlorométhyl-2 propoxy)-2 carbonyle. Notamment on fait agir un halogénure de trialkylsilane, dialkylarylsilane, alkyldiarylsilane ou de triarylsilane (par exemple le chlorure), en présence d'une base azotée comme par exemple la triéthylamine, la dipméthylaminopyridine, la pyridine ou l'imidazole, dans un solvant organique comme un amide (dimethylformamide par exemple). De préférence on opère sous atmosphère d'azote.

L'étape c) de débenzoylation de la fonction amine, en une amine primaire, peut être mise en oeuvre selon la méthode décrite par Kingston, Tetrahedron Letters, 40, 189-192 (1999) incorporée ici à titre de référence, notamment en utilisant un alcoolate de métal alcalin ou alcalino-terreux comme par exemple le méthanolate de magnésium ou l'éthanolate de magnésium. De préférence on utilise le méthanolate de magnésium.

Selon un autre mode de réalisation de l'invention l'étape c) de débenzoylation de la fonction amine, en une amine primaire, peut être également mise en oeuvre par action d'une quantité réductrice de réactif de Schwartz : Cp₂ZrHCl (hydrochlorure de zirconocène) pour lequel Cp signifie cyclopentadiènyle suivie de l'hydrolyse de l'imine obtenue. La réaction s'effectue avantageusement dans un solvant tel qu'un éther (tétrahydrofurane par exemple) à une température comprise entre 15 et 25°C, de préférence sous atmosphère d'azote.

L'hydrolyse de l'imine est conduite en milieu acide minéral-aqueux en solution dans un solvant protique choisi parmi de alcools ou des éthers. L'acide minéral peut être choisi parmi l'acide chlorhydrique ou l'acide bromhydrique. Le solvant protique peut être avantageusement choisi parmi un alcool (par exemple l'éthanol, le méthanol...), ou un éther (par exemple le tétrahydrofurane ou le dioxane). Il est entendu que lorsque l'on opère sur un dérivé de paclitaxel dont les fonctions hydroxy en -7, -10 et -2' ont été protégées par des radicaux silylés, lesdits radicaux sont éliminés simultanément à la réaction d'hydrolyse.

L'étape d) de fonctionnalisation de l'amine par un radical t.butoxycarbonyle s'effectue selon les méthodes connues de formation de carbamates à partir d'une fonction amine, qui n'altèrent pas le reste de la molécule. Notamment on peut faire agir l'anhydride de l'acide tert.butoxycarboxylique ((CH₃)₃C-O-CO)₂O sur l'amine primaire de formule générale : 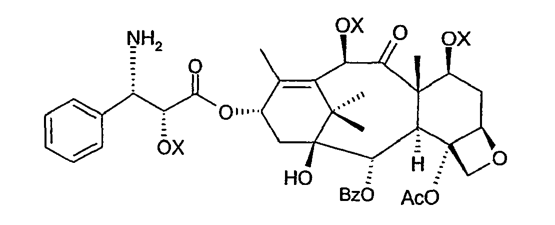 dans laquelle X représente des radicaux protecteurs tels que définis ci-dessus ou représente des atomes d'hydrogène, en opérant dans les conditions habituelles qui n'altèrent pas le reste de la molécule. Notamment on opère par addition de di-tert.butyldicarbonate à pH contrôlé de 7,5, par addition de soude, dans un alcool comme le méthanol ou en milieu hydroorganique eau/ester (eau/acétate d'éthyle par exemple), en présence d'un carbonate ou d'un bicarbonate (bicarbonate de sodium par exemple).

Selon l'invention, le paclitaxel utilisé comme produit de départ peut être avantageusement préparé à partir de la 10-déacétylbaccatine selon une réaction « one-pot » impliquant les 3 étapes successives de protection du radical hydroxy en position -7, d'acétylation du radical hydroxy en position -10, et de cristallisation du dérivé de baccatine III obtenu, suivies de la condensation de l'acide (4S,5R)-3-N-benzoyl-2RS-méthoxy-4-phényl-1,3-oxazolidine-5-carboxylique de formule : 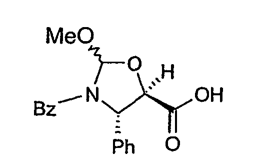 par esterification en position -13 du dérivé de 10-acétylé obtenu de formule générale : 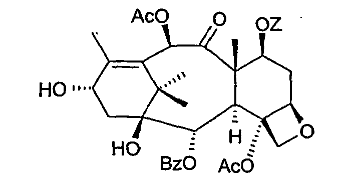 dans laquelle Z est un radical protecteur, pour obtenir un dérivé de formule générale : 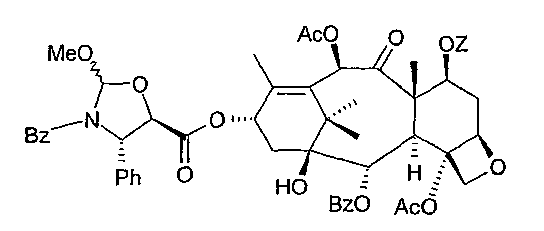 dans laquelle Z est un radical protecteur, puis ouvre l'oxazolidine de la chaîne latérale cyclique et libère simultanément le radical hydroxy en position -7 de son radical protecteur et purifie éventuellement le paclitaxel ainsi obtenu.

Le radical protecteur Z est choisi parmi des radicaux protecteurs habituellement utilisés dans la chimie des taxanes et dont la mise en place et l'élimination n'altère pas le reste de la molécule. Notamment, les radicaux protecteurs d'hydroxy tels que cités précédemment pour x.

Les conditions opératoires de la préparation du paclitaxel utilisé comme produit de départ, à partir de la 10-déacétylbaccatine, sont décrites dans la demande internationale WO 94/07878.

L'exemple suivant donné à titre non limitatif illustrent l'invention.

### Exemple

### Préparation du 10-désacétylpaclitaxel

Dans un tricol de 500 ml, le paclitaxel (6,9 g, 8,08 mmol) est solubilisé à 20°C dans 138 ml de tétrahydrofurane, sous atmosphère d'azote. Une solution d'eau oxygénée à 30% (solution refroidie à 5°C) (138 ml, 1,22 mol, >150 éq.) puis l'hydrogénocarbonate de sodium (13,2 g, 0,16 mmol, 19 éq.) sont additionnés au mélange réactionnel. Après 24 heures d'agitation, le mélange réactionnel est dilué par du dichlorométhane (500 ml). La phase organique est lavée à l'eau (500 ml). La phase aqueuse est extraite une fois avec 250 ml de dichlorométhane. La phase organique est ensuite séchée sur MgSO₄, filtrée, puis évaporée sous pression réduite. Le résidu repris dans le dichlorométhane est purifié par chromatographie (CH₂Cl₂/MeOH 96/4) pour donner 6,5 g (99%) du produit attendu.

CCM : Rf= 0,4 (CH₂Cl₂/MeOH 93/7).

### Préparation du 2',7,10-tris(triéthylsilyl)paclitaxel :

Dans un tricol de 10 ml, le 10-désacétyl paclitaxel (100 mg, 0,123 mmol) en solution dans 1 ml de diméthylformamide anhydre, sous atmosphère d'azote, est additionné de chlorure de triéthylsilane (206 µl, 1,23 mmol, 10 éq.) et d'imidazole (100 mg, 1,47 mmol, 12 éq.). Le mélange réactionnel est chauffé à 57°C pendant 7 heures. Après être revenu à température ambiante, le mélange est dilué avec 20 ml d'acétate d'éthyle. La phase organique est lavée à l'eau (20 ml), séchée sur MgSO₄, filtrée, puis évaporée sous pression réduite. Le produit brut est purifié par chromatographie (cyclohexane/acétate d'éthyle 90/10 puis 80/20). Les fractions réunies sont concentrées sous pression réduite (106 mg) pour donner 106 mg (75%) du produit attendu.

CCM : Rf= 0,3 (cyclohexane/acétate d'éthyle 80/20).

Spectre de masse : m/z = 1176,53 [M+Na]⁺

RMN ¹H (CDCl₃, 400 MHz) δ (ppm) : 0,39-0,85 (m, 27H, 9 x CH₃) ; 0,95-1,02 (m, 18H, 9 x Che₂) ; 1,18 et 1,22 (2s, 6H, CH₃-16 et CH₃-17) ; 1,68 (s, 3H, CH₃-19) ; 1,69 (s, 1H, OH-1) ; 1,86 (s, 3H, CH₃-18) ; 1,92 (m, 1H, H6-B) ; 2,13-2,39 ( m, 2H, CH₂-14) ; 2,53 (s, 3H, AcO-4) ; 2,54 (m, 1H, H6-A)°; 3,86 (d, 1H, J = 7,0Hz, H3) ; 4,20 - 4,32 (2d, 2H, J = 8,4Hz, H20) ; 4,40 (m, 1H, H7) ; 4,69 (d, 1H, J = 2,0Hz, H2') ; 4,93 (d, 1H, J = 8,1Hz, H5) ; 5,16 (s, 1H, H 10) ; 5,66 - 5,70 (m, 2H, H2 + H3') ; 6,22 (t, 1H, H13) ; 7,14 (d, 1H, J = 8,7Hz, NH) ; 7,32 - 7;43 (m, 7H, m-PhCONH, Ph) ; 7,45-7,53 (m, 3H, *m*-PhCOO et *p*-PhCONH) ; 7,60 (t, 1H, J = 7,4Hz, *p-*PhCOO) ; 7,77 (d, 2H, J = 7,1Hz, o-PhCONH) ; 8,12 (d, 2H, J = 7,2Hz, *o*-PhCOO).

### Préparation du N-débenzoyl-10-désacétylpaclitaxel :

Dans un ballon de 10 ml, à une solution de 2',7,10-tris(triéthylsilyl)paclitaxel (62 mg, 0,0537 mmol) dans 620 µl de tétrahydrofurane anhydre, sous atmosphère d'azote, est additionné le réactif de Schwartz (Cp₂ZrHCl ; 41,5 mg, 0,161 mmol, 3 éq.). Après une heure d'agitation à une température de 24°C, le mélange réactionnel est homogène. On verse le mélange après 30 minutes d'agitation supplémentaires dans 15 ml de cyclohexane refroidi (4°C). Un voile blanc se forme. Du cyclohexane froid est ajouté (10 ml). On laisse 1h30 à 4°C avant de filtrer. Le filtrat est concentré à sec puis solubilisé dans 3,1 ml de méthanol. Une solution aqueuse d'acide chlorhydrique 1N (268 µl, 0,268 mmol, 5 éq.) est ajoutée. Après 3 heures d'agitation, 2 équivalents supplémentaires d'acide chlorhydrique 1N sont ajoutés (107 µl, 0,107 mmol, 2 éq.). Le mélange est dilué avec du cyclohexane (20 ml). La phase organique est lavée à l'eau (20 ml). L'addition de 5 ml d'acétate d'éthyle permet de casser l'émulsion qui s'est formée lors du lavage. La phase organique est lavée avec 5 ml d'une solution aqueuse d'hydrogénocarbonate de sodium (5%). La phase aqueuse est extraite trois fois par 15 ml de dichlorométhane. Les phases organiques sont réunies, séchées sur MgSO₄, filtrées, puis évaporées sous pression réduite. Le brut est purifié par chromatographie (CH₂Ch/MeOH 93/7). Les fractions réunies sont concentrées sous pression réduite pour donner 27 mg (71 %) du produit attendu.

CCM : Rf = 0,3 (CH₂Cl₂/MeOH 93/7).

| | |
|---|---|
| Spectre de masse : | m/z = 708,26 [M+H]⁺ |
| | m/z = 730,23 [M+Na]⁺ |

### Préparation du docetaxel :

Dans un ballon de 10ml, le composé déprotégé (25 mg, 0,035 mmol) est solubilisé dans 3,5 ml d'acétate d'éthyle et 3,5 ml de solution aqueuse d'hydrogénocarbonate de sodium saturée. On ajoute le di-*tert*-butyl-dicarbonate (15,4 mg, 0,071 mmol, 2 éq.). Après 5h30 d'agitation, le mélange réactionnel est dilué avec 7 ml d'acétate d'éthyle. La phase organique est lavée deux fois à l'eau (7 ml). Les phases organiques sont réunies, séchées sur MgSO₄, filtrées, puis évaporées sous pression réduite. Le brut est purifié par chromatographie (CH₂Cl₂/MeOH 95/5). Les fractions réunies sont concentrées sous pression réduite pour donner 22 mg (78%) du produit attendu.

CCM : Rf = 0,2 (CH₂Cl₂/MeOH 93/7).

Spectre de masse : m/z = 830,13 [M+Na]⁺

RMN ¹H (CDCl₃, 400 MHz) δ (ppm) : 1,13 et 1,24 (2s, 6H, CH₃-16 et CH₃-17) ; 1,34 (s, 9H, *t*Bu) ; 1,74 (s, 1H, OH-1) ; 1,75 (s, 3H, CH₃-19) ; 1,85 (s, 3H, CH₃-18) ;1,81 - 1,87 (m, 1H, H6-B) ; 2,27 (d, 2H, J = 8,4Hz, CH₂-14) ; 2,37 (s, 3H, AcO-4) ; 2,54 - 2,62 (m, 1H, H6-A)°; 3,42 (s large, 1H, OH-2') ; 3,91 (d, 1H, J = 7,0Hz, H3) ; 4,18 - 4,32 (m, 4H, H20, H7 et OH-10) ; 4,62 (s, 1H, H2') ; 4,94 (d, 1H, J = 7,9Hz, H5) ; 5,21 (s, 1H, H10) ; 5,25 (d, 1H, J = 8,5Hz, H3') ; 5,45 (d, 1H, J = 9,4Hz, NH) ; 5,67 (d, 1H, J = 7,0Hz, H2) ; 6,21 (t, 1H, J = 8,6Hz, H13) ; 7,30 - 7;42 (m, 5H, Ph) ; 7,48-7,52 (m, 2H, J = 7,5Hz, *m*-PhCOO) ; 7,61 (t, 1H, J = 7,4Hz, *p*-PhCOO) ; 8,11 (d, 2H, J = 7,4Hz, o-PhCOO).

## Revendications

1. Un procédé de préparation du docétaxel à partir du paclitaxel, **caractérisé en ce que** l'on met en oeuvre les étapes suivantes :
a) déacétylation du paclitaxel de formule : 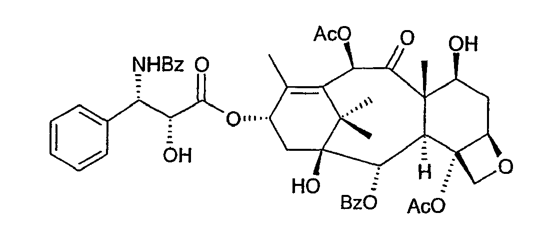
b) protection des fonctions hydroxy libres, respectivement en position -7, -10 et -2',
c) débenzoylation de l'amine en position -3', en une amine primaire dérivée de la 10-déacétylbaccatine dont les fonctions hydroxy en position -7, -10 et - 2'sont protégées,
d) fonctionnalisation de l'amine par un radical t.butoxycarbonyle pour obtenir un dérivé du docétaxel de formule générale : 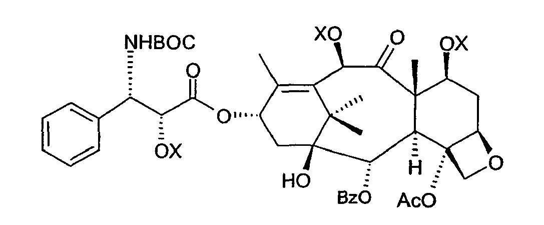 dans laquelle X représente des radicaux protecteurs ou des atomes d'hydrogène, puis, le cas échéant,
e) libération des fonctions hydroxy initialement protégées pour obtenir le docétaxel de formule :

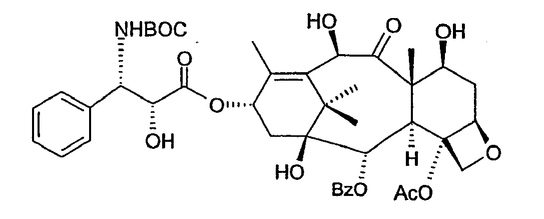

2. Un procédé selon la revendication 1, **caractérisé en ce que** le paclitaxel utilisé comme produit de départ est préparé à partir de la 10-déacétylbaccatine selon une réaction « one-pot » impliquant les 3 étapes successives de protection du radical hydroxy en position -7, d'acétylation du radical hydroxy en position -10, et de cristallisation du dérivé de baccatine III obtenu, suivies de la condensation de l'acide (4S,5R)-3-N-benzoyl-2RS-méthoxy-4-phényl-1,3-oxazolidine-5-carboxylique de formule : 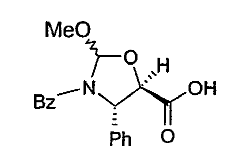 par estérification en position -13 du dérivé de 10-acétylé obtenu, de formule générale : 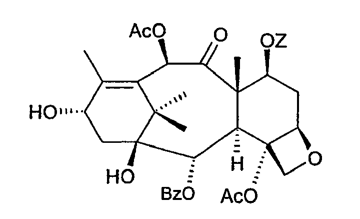 dans laquelle Z est un radical protecteur, pour obtenir un dérivé de formule générale : 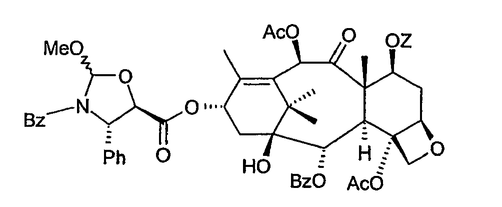 dans laquelle Z est un radical protecteur, puis ouvre l'oxazolidine de la chaîne latérale cyclique et libère simultanément le radical hydroxy en position -7 de son radical protecteur et purifie éventuellement le paclitaxel ainsi obtenu.

3. Un procédé selon la revendication 1 ou 2, **caractérisé en ce que** la déacétylation s'effectue par action d'un peroxyde en milieu basique.

4. Un procédé selon la revendication 3, **caractérisé en ce que** le peroxyde est choisi parmi le peroxyde d'hydrogène, le peroxyde de tert.butyle l'acide métachloroperbenzoïque ou un sel de l'acide monoperoxyphtalique.

5. Un procédé selon l'une des revendications 3 ou 4, **caractérisé en ce que** la base est choisie parmi les carbonates ou les hydroxydes de métaux alcalins ou alcalino-terreux.

6. Un procédé selon la revendication 5, **caractérisé en ce que** la base est choisie parmi le carbonate de calcium, le bicarbonate de sodium, le carbonate de sodium, le carbonate de potassium, le carbonate de baryum, le carbonate de césium, l'hydroxyde de sodium, de potassium, de lithium, de baryum, de calcium ou de magnésium.

7. Un procédé selon l'une des revendications 1 à 6, **caractérisé en ce que** les radicaux protecteurs d'hydroxy sont choisis parmi les radicaux silylés ou trichloroalkyloxycarbonyle.

8. Un procédé selon la revendication 7, **caractérisé en ce que** les radicaux silylés sont choisis parmi les groupements trialkylsilyle, dialkylarylsilyle, alkyldiarylsilyle ou triarylsilyle dans lesquelles les radicaux alkyles contiennent 1 à 4 C en chaîne droite ou ramifiée et aryle représente phényle, de préférence triéthylsilyle ou triméthylsilyle et **en ce que** les radicaux trichloroalkyloxycarbonyle sont choisis parmi les groupements trichloréthoxycarbonyle ou trichlorométhylpropoxycarbonyle.

9. Un procédé selon l'une des revendications 1 à 8, **caractérisé en ce que** l'étape c) de débenzoylation de la fonction amine, en une amine primaire, est mise en oeuvre par action d'un alcoolate de métal alcalin ou alcalino-terreux.

10. Un procédé selon la revendication 9, **caractérisé en ce que** l'alcoolate est choisi parmi le méthanolate de magnésium ou l'éthanolate de magnésium.

11. Un procédé selon l'une des revendications 1 à 8, **caractérisé en ce que** l'étape c) de débenzoylation de la fonction amine, en une amine primaire, est mise en oeuvre par action d'une quantité réductrice de réactif de Schwartz : Cp₂ZrHCl pour lequel Cp signifie cyclopentadiènyle suivie de l'hydrolyse de l'imine obtenue.

12. Un procédé selon la revendication 11, **caractérisé en ce que** l'hydrolyse de l'imine est conduite en milieu acide minéral-aqueux en solution dans un solvant protique choisi parmi de alcools ou des éthers.

13. Un procédé selon la revendication 12, **caractérisé en ce que** l'acide minéral est choisi parmi l'acide chlorhydrique ou bromhydrique, et le solvant protique est choisi parmi l'éthanol, le méthanol, le tétrahydrofurane ou le dioxane.

14. Un procédé selon l'une des revendications 1 à 13 **caractérisé en ce que** l'étape d) de fonctionnalisation de l'amine par un radical t.butoxycarbonyle s'effectue selon les méthodes connues de formation de carbamates à partir d'une fonction amine, qui n'altèrent pas le reste de la molécule.

15. Un procédé selon la revendication 14, **caractérisé en ce que** l'on fait agir l'anhydride de l'acide tert.butoxycarboxylique ((CH₃)₃C-O-CO)₂O sur l'amine primaire de formule générale : 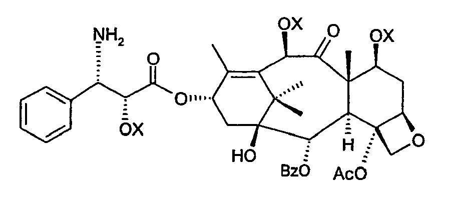 dans laquelle X représente des radicaux protecteurs ou des atomes d'hydrogène.

16. Un procédé selon la revendication 15, **caractérisé en ce que** la réaction est mise en oeuvre à pH contrôlé de 7,5, en solution dans un alcool.

17. Un procédé selon la revendication 15, **caractérisé en ce que** la réaction est mise en oeuvre en milieu hydroorganique eau/ester (eau/acétate d'éthyle par exemple), en présence d'un carbonate ou d'un bicarbonate.

18. Un procédé selon l'une des revendications 1 à 13, **caractérisé en ce que** l'on désacétyle le paclitaxel, sous atmosphère d'azote, par l'eau oxygénée à 30% en présence d'hydrogénocarbonate de sodium, à 20°C dans le tétrahydrofurane, puis on protège les positions -7, -10 et -2' du 10-désacétylpaclitaxel par des radicaux triéthylsilyle par action du chlorure de triéthylsilane dans le diméthyJforn1amide en présence d'imidazole, puis on débenzoyle le 2',7,10-tris(triéthylsilyl)paclitaxel par action du réactif de Schwartz (Cp₂ZrHCl) dans le tétrahydrofurane anhydre, sous atmosphère d'azote, à une tempérture de 24°C et traite par l'acide chlorhydrique 1N l'imine obtenue, puis prépare le docétaxel à partir du composé déprotégé obtenu, par traitement par le di-tert-butyl-dicarbonate en présence d'une solution aqueuse d'hydrogénocarbonate de sodium saturée, dans l'acétate d'éthyle.

## Claims

1. A process for preparing docetaxel from paclitaxel, **characterized in that** the following steps are performed:
a) deacetylation of paclitaxel of formula: 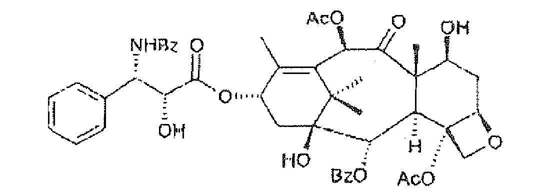
b) protection of the free hydroxyl functions, in positions -7, -10 and -2', respectively,
c) debenzoylation of the amine in position -3' to a primary amine derived from 10-deacetylbaccatin whose hydroxyl functions in positions -7, -10 and -2' are protected,
d) functionalization of the amine with a t-butoxycarbonyl radical to obtain a docetaxel derivative of general formula: 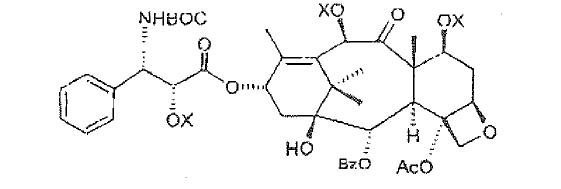 in which X represents protecting radicals or hydrogen atoms, and then, where appropriate,
e) release of the initially protected hydroxyl functions to obtain docetaxel of formula:

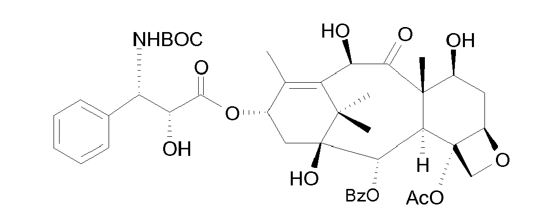

2. Process as claimed in claim 1, **characterized in that** the paclitaxel used as starting material is prepared from 10-deacetylbaccatin according to a "one-pot" reaction involving the three successive steps of protecting the hydroxyl radical in position -7, of acetylation of the hydroxyl radical in position -10, and of crystallization of the baccatin III derivative obtained, followed by condensation of (4S,5R)-3-N-benzoyl-2RS-methoxy-4-phenyl-1,3-oxazolidine-5-carboxylic acid of formula: 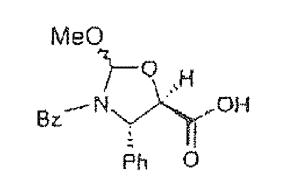 by esterification in position -13 of the 10-acetyl derivative obtained of general formula: 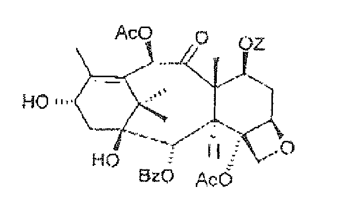 in which Z is a protecting radical, to obtain a derivative of general formula: 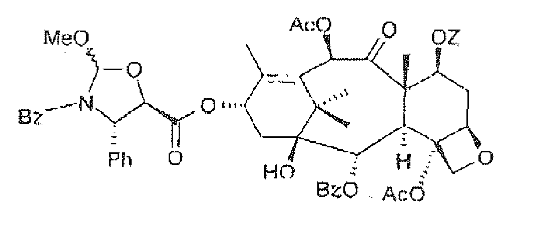 in which Z is a protecting radical, followed by opening the oxazolidine of the cyclic side chain and simultaneous release of the hydroxyl radical in position -7 from its protecting radical and optional purification of the paclitaxel thus obtained.

3. Process as claimed in claim 1 or 2, **characterized in that** the deacetylation is performed via the action of a peroxide in basic medium.

4. Process as claimed in claim 3, **characterized in that** the peroxide is chosen from hydrogen peroxide, tert-butyl peroxide, meta-chloroperbenzoic acid and a monoperoxyphthalic acid salt.

5. Process as claimed in either of claims 3 and 4, **characterized in that** the base is chosen from alkali metal or alkaline-earth metal carbonates or hydroxides.

6. Process as claimed in claim 5, **characterized in that** the base is chosen from calcium carbonate, sodium bicarbonate, sodium carbonate, potassium carbonate, barium carbonate, caesium carbonate, sodium hydroxide, potassium hydroxide, lithium hydroxide, barium hydroxide, calcium hydroxide and magnesium hydroxide.

7. Process as claimed in one of claims 1 to 6, **characterized in that** the hydroxyl-protecting radicals are chosen from silyl and trichloroalkyloxycarbonyl radicals.

8. Process as claimed in claim 7, **characterized in that** the silyl radicals are chosen from trialkylsilyl, dialkylarylsilyl, alkyldiarylsilyl and triarylsilyl groups in which the alkyl radicals contain 1 to 4 C in a straight or branched chain and aryl represents phenyl, preferably triethylsilyl or trimethylsilyl, and **in that** the trichloroalkyloxycarbonyl radicals are chosen from trichloroethoxycarbonyl and trichloromethylpropoxycarbonyl groups.

9. Process as claimed in one of claims 1 to 8, **characterized in that** step c) of debenzoylation of the amine function, to a primary amine, is performed via the action of an alkali metal or alkaline-earth metal alkoxide.

10. Process as claimed in claim 9, **characterized in that** the alkoxide is chosen from magnesium methoxide and magnesium ethoxide.

11. Process as claimed in one of claims 1 to 8, **characterized in that** step c) of debenzoylation of the amine function, to a primary amine, is performed via the action of a reductive amount of Schwartz reagent: Cp₂ZrHCl for which Cp means cyclopentadienyl, followed by hydrolysis of the imine obtained.

12. Process as claimed in claim 11, **characterized in that** the hydrolysis of the imine is performed in a medium of aqueous mineral acid dissolved in a protic solvent chosen from alcohols and ethers.

13. Process as claimed in claim 12, **characterized in that** the mineral acid is chosen from hydrochloric acid and hydrobromic acid, and the protic solvent is chosen from ethanol, methanol, tetrahydrofuran and dioxane.

14. Process as claimed in one of claims 1 to 13, **characterized in that** step d) of functionalization of the amine with a t-butoxycarbonyl radical is performed according to the known methods for formation of carbamates from an amine function, which do not impair the rest of the molecule.

15. Process as claimed in claim 14, **characterized in that** tert-butoxycarboxylic acid anhydride ((CH₃)₃C-O-CO)₂O is reacted with the primary amine of general formula: 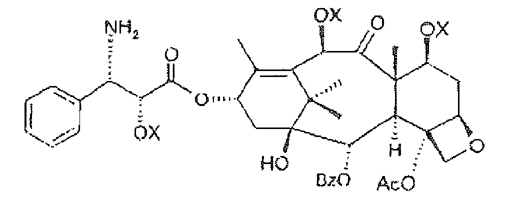 in which X represents protecting radicals or hydrogen atoms.

16. Process as claimed in claim 15, **characterized in that** the reaction is performed at a controlled pH of 7.5, in solution in an alcohol.

17. Process as claimed in claim 15, **characterized in that** the reaction is performed in hydro-organic water/ester medium (for example water/ethyl acetate), in the presence of a carbonate or a bicarbonate.

18. Process as claimed in one of claims 1 to 13, **characterized in that** paclitaxel is deacetylated, under nitrogen atmosphere, with aqueous 30% hydrogen peroxide solution in the presence of sodium hydrogencarbonate, at 20°C in tetrahydrofuran, and positions -7, -10 and -2' of the 10-deacetylpaclitaxel are then protected with triethylsilyl radicals via the action of triethylsilane chloride in dimethylformamide in the presence of imidazole, the 2',7,10-tris(triethylsilyl)paclitaxel is then debenzoylated via the action of Schwartz reagent (Cp₂ZrHCl) in anhydrous tetrahydrofuran, under a nitrogen atmosphere, at a temperature of 24°C, and the imine obtained is treated with 1N hydrochloric acid, and docetaxel is then prepared from the deprotected compound obtained, by treatment with di-*tert*-butyl dicarbonate in the presence of saturated aqueous sodium hydrogencarbonate solution, in ethyl acetate.

## Patentansprüche

1. Verfahren zur Herstellung von Docetaxel aus Paclitaxel, **dadurch gekennzeichnet, dass** die folgenden Schritte durchgeführt werden:
a) Deacetylierung von Paclitaxel der Formel: 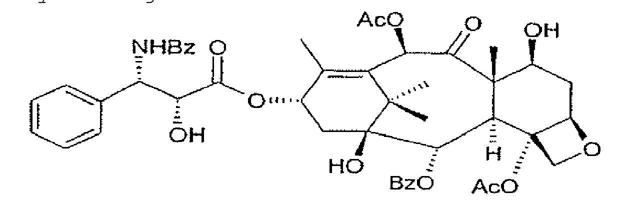
b) Schützen der freien funktionellen Hydroxygruppen, an den Positionen -7, -10 beziehungsweise -2',
c) Debenzylierung des Amins an Position -3' zu einem primären Amin, das sich vom 10-Deacetylbaccatin ableitet, wobei dessen funktionelle Hydroxygruppen an den Positionen -7, -10 und - 2' geschützt sind,
d) Funktionalisierung des Amins durch eine t-Butoxycarbonylgruppe, um ein Docetaxel-Derivat der folgenden allgemeinen Formel zu erhalten: 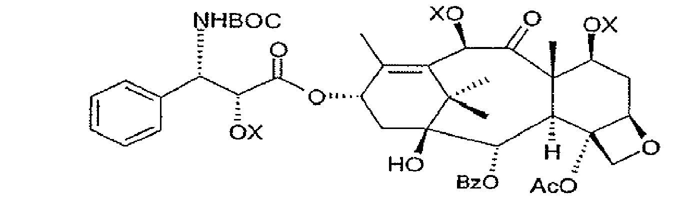 in welcher X für Schutzgruppen oder Wasserstoffatome steht, woraufhin gegebenenfalls e) die Schutzgruppen von den funktionellen Hydroxygruppen entfernt werden, um Docetaxel der folgenden Formel zu erhalten:

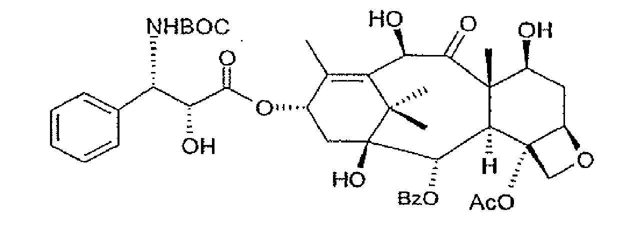

2. Verfahren nach Anspruch 1, **dadurch gekennzeichnet, dass** das Paclitaxel, welches als Ausgangsstoff verwendet wird, ausgehend von 10-Deacetylbaccatin gemäß einer "Eintopfreaktion" hergestellt wird, an welcher die nacheinander durchgeführten Schritte des Schützens der Hydroxygruppe an Position -7, der Acetylierung der Hydroxygruppe an Position -10 und der Kristallisation des erhaltenen Baccatin-III-Derivats beteiligt sind, woraufhin die (4S,5R)-3-N-Benzoyl-2RS-metboxy-4-phenyl-1,3-oxazoliden -5-carbonsäure der Formel: 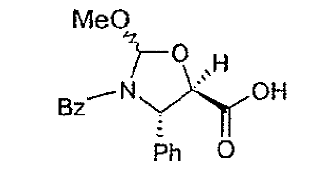 eine Kondensationsreaktion erfährt, indem sie an Position -13 des erhaltenen 10-Acetylderivats der folgenden allgemeinen Formel: 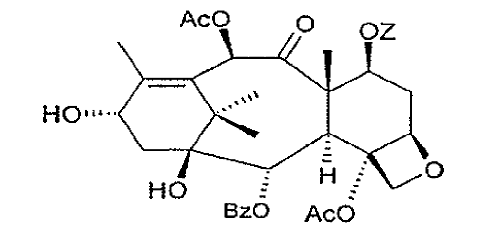 in welcher Z eine Schutzgruppe ist, eine Esterbindung eingeht, um ein Derivat der folgenden allgemeinen Formel zu erhalten: 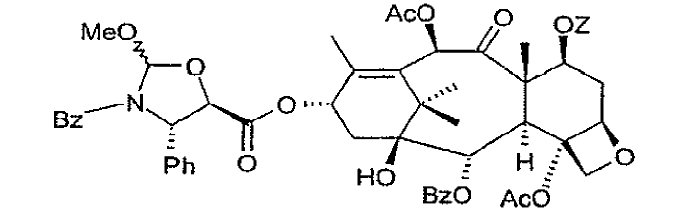 in welcher Z eine Schutzgruppe ist, woraufhin das Oxazolidin der zyklischen Seitenkette geöffnet und gleichzeitig die Schutzgruppe von der Hydroxygruppe an Position -7 entfernt wird und das auf diese Weise erhaltene Paclitaxel möglicherweise aufgereinigt wird.

3. Verfahren nach Anspruch 1 oder 2, **dadurch gekennzeichnet, dass** die Deacetylierung durch die Einwirkung eines Peroxids in basischem Milieu erfolgt.

4. Verfahren nach Anspruch 3, **dadurch gekennzeichnet, dass** das Peroxid aus Wasserstoffperoxid, tert.-Butylperoxid, meta-Chlorperbenzoesäure oder einem Salz der Monoperoxyphthalsäure gewählt ist.

5. Verfahren nach einem der Ansprüche 3 oder 4, **dadurch gekennzeichnet, dass** die Base aus den Carbonaten oder den Hydroxiden von Alkali- oder Erdalkalimetallen gewählt ist.

6. Verfahren nach Anspruch 5, **dadurch gekennzeichnet, dass** die Base aus Calciumcarbonat, Natriumhydrogencarbonat, Natriumcarbonat, Kaliumcarbonat, Bariumcarbonat, Cäsiumcarbonat, Natrium-, Kalium-, Lithium-, Barium, Calcium- oder Magnesiumhydroxid gewählt ist.

7. Verfahren nach einem der Ansprüche 1 bis 6, **dadurch gekennzeichnet, dass** die Hydroxyschutzgruppen aus den silylartigen oder den Trichloralkyloxycarbonylgruppen gewählt sind.

8. Verfahren nach Anspruch 7, **dadurch gekennzeichnet, dass** die silyartigen Gruppen aus den Gruppen Trialkylsilyl, Dialkylarylsilyl, Alkyldiarylsilyl oder Triarylsilyl, in welchen die Alkylgruppen 1 bis 4 C in geradkettiger oder verzweigter Anordnung enthalten und Aryl für Phenyl steht, vorzugsweise aus Triethylsilyl oder Trimethylsilyl, gewählt sind und dass die Trichloralkyloxycarbonylgruppen aus den Gruppen Trichlorethoxycarbonyl oder Trichlormethylpropoxycarbonyl gewählt sind.

9. Verfahren nach einem der Ansprüche 1 bis 8, **dadurch gekennzeichnet, dass** der Schritt c) der Debenzylierung der funktionellen Amingruppe zu einem primären Amin durch Einwirkung eines Alkoholats eines Alkali- oder Erdalkalimetalls erfolgt.

10. Verfahren nach Anspruch 9, **dadurch gekennzeichnet, dass** das Alkohol aus Magnesiummethanolat oder Magnesiumethanolat gewählt ist.

11. Verfahren nach einem der Ansprüche 1 bis 8, **dadurch gekennzeichnet, dass** der Schritt c) der Debenzylierung der funktionellen Amingruppe zu einem primären Amin durch Einwirkung einer reduzierenden Menge an Schwartz-Reagenz: Cp₂ZrHCl, wobei Cp Cyclopentadienyl bedeutet, mit anschließender Hydrolyse des erhaltenen Imins erfolgt.

12. Verfahren nach Anspruch 11, **dadurch gekennzeichnet, dass** die Hydrolyse des Imins einem mit mineralsauer-wässrigen Milieu in einem protischen Lösemittel, das aus den Alkoholen oder den Ethern gewählt ist, erfolgt.

13. Verfahren nach Anspruch 12, **dadurch gekennzeichnet, dass** die Mineralsäure aus Salzsäure oder Bromwasserstoffsäure gewählt ist und das protische Lösemittel aus Ethanol, Methanol, Tetrahydrofuran oder Dioxan gewählt ist.

14. Verfahren nach einem der Ansprüche 1 bis 13, **dadurch gekennzeichnet, dass** der Schritt d) der Funktionalisierung des Amins durch eine t-Butoxycarbonylgruppe gemäß den bekannten Verfahren zur Bildung von Carbamaten aus einer funktionellen Amingruppe erfolgt, wobei diese den Rest den Moleküls unverändert lassen.

15. Verfahren nach Anspruch 14, **dadurch gekennzeichnet, dass** das Anhydrid der tert.-Butoxycarbonsäure [(CH₃)₃C-O-OO]₂O mit dem primären Amin der folgenden allgemeinen Formel: 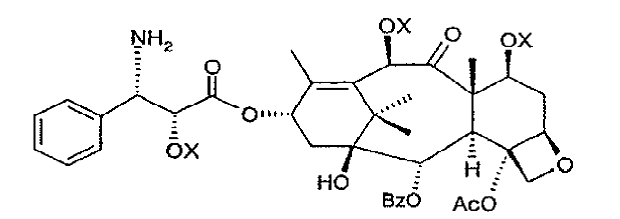 in welcher X für Schutzgruppen oder Wasserstoffatome steht, umgesetzt wird.

16. Verfahren nach Anspruch 15, **dadurch gekennzeichnet, dass** die Reaktion in einer alkoholischen Lösung bei einem pH-Wert durchgeführt wird, der gezielt bei 7,5 gehalten wird.

17. Verfahren nach Anspruch 15, **dadurch gekennzeichnet, dass** die Reaktion in einem wässrig-organischem Wasser/Ester-Milieu durchgeführt wird (Wasser/Ethylacetat beispielsweise), in Gegenwart eines Carbonats oder eines Hydrogencarbonats.

18. Verfahren nach einem der Ansprüche 1 bis 13, **dadurch gekennzeichnet, dass** das Paclitaxel unter Stickstoffatmosphäre deacetyliert wird, und zwar durch 30%iges Wasserstoffperoxid in Gegenwart von Natriumhydrogencarbonat, bei 20 °C in Tetrahydrofuran, woraufhin an den Positionen -7, -10 und -2' des 10-Deacetyl-paclitaxels Triethylsilyl-Schutzgruppen durch Einwirkung von Triethylsilanchlorid in Dimethylformamid in Gegenwart von Imidazol eingeführt werden, woraufhin das 2',7,10-tris-(Triethylsilyl)-paclitaxel durch Einwirkung des Schwartz-Reagenzes (Cp₂ZrHCl) in wasserfreiem Tetrahydrofuran, unter Stickstoffatmosphäre bei einer Temperatur von 24 °C, debenzyliert wird und das erhaltene Imin mit Salzsäure 1N behandelt wird, woraufhin aus der erhaltenen Verbindung, die keinerlei Schutzgruppen aufweist, durch Behandlung mit di-tert.-Butyldicarbonat in Gegenwart einer wässrigen gesättigten Natriumhydrogencarbonatlösung das Docetaxel gewonnen wird, in Ethylacetat.
